# EUROPEAN PATENT APPLICATION

(11) **EP 4 444 041 A1**
(43) Date of publication of application: **09.10.2024**
(21) Application number: 22928864.2
(22) Date of filing: 31.10.2022
(51) Int. Cl.: H05B 41/30, A61L 2/10, H01J 61/10, H01J 61/16, H01J 61/40, H01J 65/00

(54) **LIGHT SOURCE DEVICE**

(30) Priority: 28.02.2022 JP 2022030348
(71) Applicant: Ushio Denki Kabushiki Kaisha, Tokyo 100-8150 (JP)
(72) Inventor: FUKUDA, Minoru, Tokyo 100-8150 (JP)
(74) Representative: Maiwald GmbH
(86) International application number: PCT/JP2022/040587
(87) International publication number: WO 2023/162346

(57) **Abstract**

An object of the present invention is to provide a light source device having a novel structure and including an ultraviolet radiation lamp that emits light by dielectric barrier discharge and a lighting circuit. The light source device includes an ultraviolet radiation lamp (1) and a lighting circuit (S). The lamp (1) includes: a substantially rod-shaped light-emitting tube (11) in which light-emitting gas containing at least a halogen gas is sealed; and a pair of electrodes (12, 13) for generating discharge plasma (14) inside the light-emitting tube (11) in a direction in which the light-emitting tube extends, and the lighting circuit (S) constitutes a flyback circuit and controls a width of pulse voltage applied to the electrodes to be 450 ns or more to 1000 ns or less.

## Description

### TECHNICAL FIELD

The present invention relates to a light source device. In particular, the present invention relates to a light source device including an ultraviolet radiation lamp that emits light by dielectric barrier discharge and a lighting circuit.

### BACKGROUND ART

In recent years, a sterilization device using an ultraviolet radiation lamp that emits light by dielectric barrier discharge is known. This ultraviolet radiation lamp generates so-called dielectric barrier discharge by a pair of electrodes disposed with a light-emitting tube as a dielectric material interposed therebetween, and emits light corresponding to a light-emitting gas sealed inside the tube. In a case where Kr (krypton) and Cl (chlorine) are sealed as the light-emitting gas, ultraviolet light having a single peak at a wavelength of 222 nm is generated.

Recently, this type of ultraviolet radiation lamp is expected as a light source that can inactivate microorganisms and viruses while suppressing adverse effects on humans and animals, and is expected to be used in various scenes such as facilities where people frequently gather, such as medical facilities, schools, and government offices, and vehicles such as automobiles, trains, buses, airplanes, and ships. Therefore, the light source device is also strongly required to respond to such a demand and to be developed to a miniaturized device.

### PRIOR ART DOCUMENT

### PATENT DOCUMENTS

Patent Document 1: JP-B2-5979016
Patent Document 2: JP-B2-6025756

### SUMMARY OF THE INVENTION

### PROBLEMS TO BE SOLVED BY THE INVENTION

An object of the present invention is to provide a light source device having a novel structure and including an ultraviolet radiation lamp that emits light by dielectric barrier discharge and a lighting circuit.

### MEANS FOR SOLVING THE PROBLEMS

A light source device according to the present invention includes an ultraviolet radiation lamp that emits light by dielectric barrier discharge and a lighting circuit of this ultraviolet radiation lamp. The ultraviolet radiation lamp includes: a substantially rod-shaped light-emitting tube in which light-emitting gas containing at least a halogen gas is sealed; and a pair of electrodes for generating discharge plasma inside the light-emitting tube in a direction in which the light-emitting tube extends, and the lighting circuit constitutes a flyback circuit and controls a width of pulse voltage applied to the electrodes to be 450 nanoseconds (ns) or more to 1000 ns or less.

When the pulse width of the voltage applied to the dielectric barrier discharge lamp becomes too narrow, power supply to the lamp becomes insufficient, and in some cases, the dielectric barrier discharge lamp is not turned on easily. Specifically, when the applied pulse width becomes too narrow, a pulse voltage value that can be applied is greatly reduced due to the influence of parasitic elements in the power supply line and the light-emitting tube, and the lighting operation of the lamp becomes difficult.

Further, in a case where a halogen gas having high electron affinity is contained in the light-emitting gas, the lamp has characteristics that initial discharge is more difficult to occur than in an ultraviolet radiation lamp in which the halogen gas is not contained in the light-emitting gas. Therefore, when the pulse width is too narrow, there is a high possibility that the initial discharge cannot be maintained or does not occur. Note that in a case where a high voltage is applied in order to generate the initial discharge, there is a possibility that the luminance efficiency rather deteriorate, or the life of the light-emitting tube is shortened. Note that the discharge plasma generated in the light-emitting tube in which the light-emitting gas is sealed disappears in about several tens of nanoseconds unless discharge is maintained.

In consideration of the above content and deterioration of the voltage pulse due to the parasitic element, the pulse width of the voltage applied between the electrodes is expected to be at least about 300 ns in order to reliably generate the ultraviolet light in the light-emitting tube. In addition, considering variations due to the length or the like of the power supply line, a point that lighting up at the time of starting is difficult, and the like, it can be seen that the pulse width required to realize reliable starting is 450 ns or more.

Further, the lighting circuit controls the width of the pulse voltage applied to the pair of electrodes to be 800 ns or less.

In addition, the ultraviolet radiation lamps are sealed with krypton and chlorine, or krypton and bromine.

Furthermore, the ultraviolet radiation lamp has a filter that emits light having a wavelength of 200 nm ~ 230 nm and cuts off radiation light of UVC having other wavelengths.

### EFFECT OF THE INVENTION

The light source device according to the present invention can stabilize discharge even in a lamp form in which discharge plasma is generated in the longitudinal direction of the light-emitting tube by controlling the half-value width with respect to the peak voltage value of the peak pulse voltage waveform applied to the pair of electrodes of the ultraviolet radiation lamp to be 1000 ns or less by using the flyback lighting circuit.

Moreover, in the light source device according to the present invention, by the flyback lighting circuit controlling the half-value width with respect to the peak voltage value of the peak pulse voltage waveform applied to the pair of electrodes of the ultraviolet radiation lamp to be 800 ns or less, the radiation efficiency of the ultraviolet light can be enhanced.

### BRIEF DESCRIPTION OF THE DRAWINGS

Fig. 1 illustrates an overall configuration of a light source device according to the present invention.
Fig. 2 illustrate enlarged views of an excimer lamp according to the present invention.
Fig. 3 illustrate enlarged views of an excimer lamp according to the present invention.
Fig. 4 illustrates a waveform of a voltage applied to the lamp of the light source device according to the present invention.
Fig. 5 illustrates an experimental result of the light source device according to the present invention.
Fig. 6 illustrates an experimental result of the light source device according to the present invention.

### MODE FOR CARRYING OUT THE INVENTION

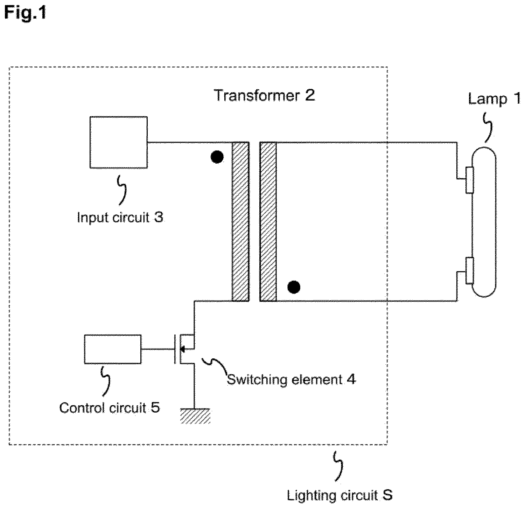

Fig. 1 illustrates an overall configuration of a light source device according to the present invention. The light source device includes an ultraviolet radiation lamp 1(hereinafter, also simply referred to as a "lamp") and a lighting circuit S. Both electrodes of the lamp 1 are electrically connected to a secondary winding of a transformer 2. An input circuit 3 to which power is supplied from a commercial power supply or a direct current power supply is connected to a primary winding of the transformer 2. A switching element 4 such as a FET element is connected to the other end of the primary winding of the transformer 2, and a control circuit 5 is connected to a gate of the switching element 4. This circuit is generally called a boost flyback circuit, and a high voltage waveform is periodically generated on the secondary winding of the transformer 2 corresponding to an off timing of the switching element 4. The lighting circuit S includes the transformer 2, the input circuit 3, the switching element 4, and the control circuit 5.

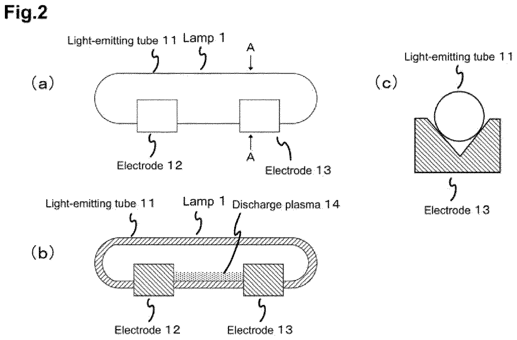

Fig. 2 illustrates enlarged views of the ultraviolet radiation lamp according to the present invention. (a) of Fig. 2 illustrates an external view of the lamp, (b) of Fig. 2 illustrates an internal structure of the lamp, and (c) of Fig. 2 illustrates a A-A cross-sectional view in (a). The lamp 1 includes a light-emitting tube 11 having a rod shape as a whole, and a pair of electrodes 12 and 13 is provided at both ends thereof. The light-emitting tube 11 is made of quartz glass which is a dielectric material, and xenon gas is sealed therein as a discharge gas. When a voltage is applied to both electrodes, plasma 14 is generated inside the light-emitting tube 11 as illustrated in (b) of Fig. 2 to form the discharge column. By this discharge, the sealed gas is brought into an excimer state to generate UVC light having a wavelength of 222 nm. The ultraviolet radiation lamp according to the present invention performs light emission using dielectric barrier discharge, and is also referred to as a dielectric barrier discharge lamp or an excimer lamp.

Here, in the present embodiment, a V-shaped holding base that holds the lamp 1 constitutes the electrode. Therefore, a lamp structure is formed by installing the light-emitting tube 11 on the electrode 12 and the electrode 13. Discharge plasma 14 is generated so as to extend in the longitudinal direction of the lamp 1, but the discharge tends to be unstable depending on the contact relationship between the electrode and the light-emitting tube. However, the present invention is improved by devising a voltage waveform to be applied to the lamp as described later.

Further, in a case where the light source device according to the present invention is used as a sterilization device, it is desirable that the light source device is a lamp that emits so-called UVC light (200 nm ~ 280 nm or less). In particular, in a case where bromine and krypton are sealed in the light-emitting tube, UVC of a spectrum showing a peak value at a wavelength of 208 nm is emitted, and in a case where chlorine and krypton are sealed therein, UVC of a spectrum showing a peak value at a wavelength of 222 nm is emitted. The light having a wavelength of 200 nm ~ 230 nm does not adversely affect the cell nucleus of a human body or an animal even when the human body or the animal is irradiated with the light. Therefore, by using these ultraviolet radiation lamps, a light source device that can inactivate viruses and microorganisms can be provided while the influence on the human body is avoided. In addition, the influence on the human body can be more reliably avoided by providing a filter that cuts the UVC light having a wavelength other than 200 nm ~ 230 nm. Regarding the lamp 1, as a numerical example, the light-emitting tube 11 has a rated power of 12 W, a total length of 40 mm, and a light-emitting tube diameter ϕ of 6 mm.

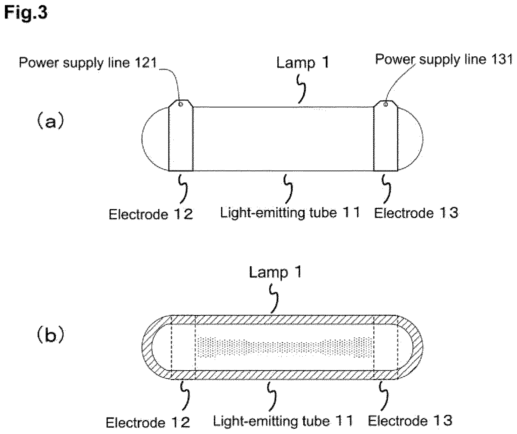

Fig. 3 illustrates enlarged views of another embodiment of an ultraviolet radiation lamp according to the present invention. (a) of Fig. 3 illustrates an external view of the lamp, and (b) of Fig. 3 illustrates an internal structure of the lamp. This lamp has a different electrode structure as compared with the lamp illustrated in Fig. 2. That is, an electrode 12 and an electrode 13 are each wound around a light-emitting tube 11 in a form of a strip-shaped thin metal piece. Power supply lines 121 and 131 electrically connected to a transformer are connected to the respective electrodes. In this structure, there is a case where the contact state between the electrode and the light-emitting tube becomes partially non-uniform, and the electrode possibly becomes partially separated from the surface of the light-emitting tube. Therefore, the discharge tends to be unstable. However, the present invention is improved by devising a voltage waveform to be applied to the lamp as described later. Note that, although a print electrode is adopted in some cases as a light source for copier, in the present embodiment, the electrode structures illustrated in Figs. 2 and 3 are adopted in order to achieve a small and inexpensive lamp.

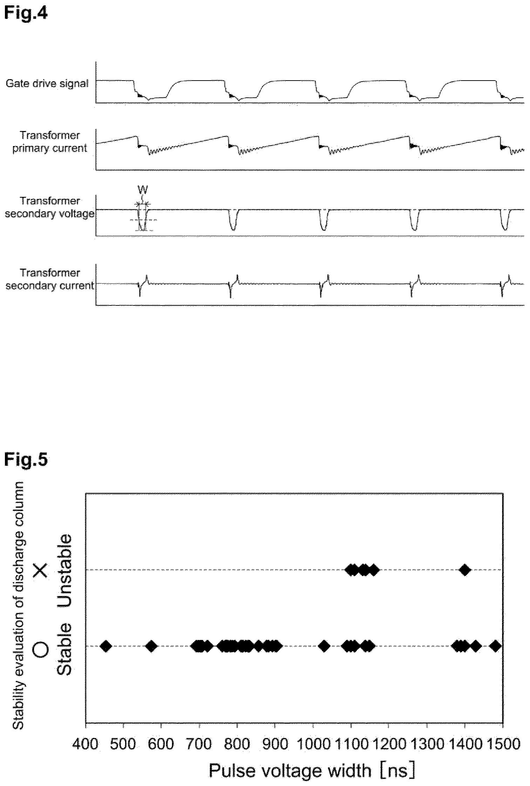

Fig. 4 illustrates a gate drive signal, a primary current waveform of the transformer, a secondary voltage waveform of the transformer, and a secondary current waveform of the transformer in the light source device according to the present invention. Because a flyback circuit is constituted, when the gate drive signal supplied to the switching element is turned off, a single-peak voltage waveform is generated in the secondary winding of the transformer 2, and a ramp current also flows. Thereafter, the same waveform is repeatedly and periodically generated in accordance with the on/off timing of the gate drive signal.

Fig. 5 illustrates experimental results on stability of a discharge column. That is, the drawing illustrates the results of the experiment on a pulse width of the voltage applied to the lamp and fluttering of the discharge column. The vertical axis indicates the fluttering (positional stability) of the discharge column, and the horizontal axis indicates the pulse voltage width (ns: nanosecond). In the experiment, the lamp having the structure illustrated in Fig. 2 was used, and the discharge state of the discharge column was visually observed for about ten seconds, and the one in which the discharge continued to be stable within the observation time was determined as "stable", and the one in which the discharge continued to fluctuate unstably within the observation time was determined as "unstable". Note that the pulse voltage width is a half-value width with respect to the peak voltage value. Furthermore, the pulse voltage width is adjusted by changing the inductance of the secondary winding of the transformer. In this verification, the stability of the discharge column was evaluated in a pulse voltage width range of 450 ns or more and 1500 ns or less.

From the experimental results shown in Fig. 5, when the pulse voltage width exceeded 1000 ns, there were cases where the discharge column during lighting continued to be stable and fluctuated unstably. On the other hand, when the pulse voltage width was 1000 ns or less, the discharge column during lighting was stable. As a result, in the lamp structure in which the discharge plasma is formed in the extending direction of the light-emitting tube as in the present invention, in the method in which the voltage is supplied by the flyback method, it has been confirmed that the discharge column at the time of lighting is satisfactorily stabilized by setting the pulse voltage width to 1000 ns or less. Note that, when the pulse voltage width was 457 ns, the discharge during lighting was substantially stable, but when the pulse voltage width was less than 450 ns, the lighting operation of the lamp was not stable and difficult. The reason for this is presumed to be that, as described above, when the pulse voltage width becomes too narrow, the influence from other devices, members, and the like becomes significant, and the peak value is greatly reduced in some cases.

As a method of adjusting the pulse voltage width, in addition to a method of adjusting the pulse voltage width by adjusting the inductance of the transformer, there are a method by a signal applied to the switching element, a method by adjusting the core gap of the transformer.

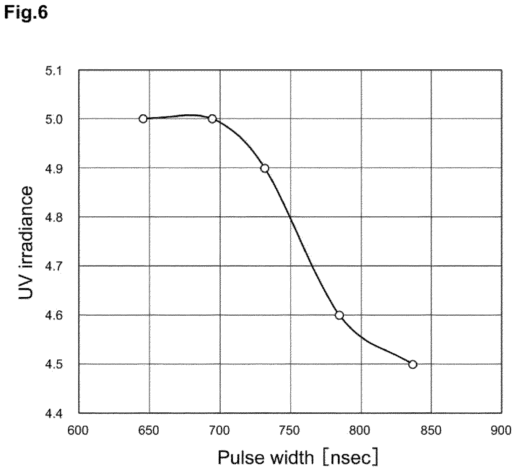

Fig. 6 illustrates experimental results on UV irradiance values. That is, the drawing illustrates the experiment on the pulse width of the voltage applied to the lamp and the luminous efficiency. The vertical axis represents an integral value of a wavelength of 200 nm ~ 230 nm, and the horizontal axis represents a pulse voltage width (ns: nanosecond). Note that, similarly to the experiment illustrated in Fig. 5, the pulse voltage width is a half-value width with respect to the peak voltage value. Furthermore, the pulse voltage width is adjusted by changing the inductance of the secondary winding of the transformer.

Fig. 6 illustrates a graph of the experimental results, and describing as numerical values, the UV irradiance is 4.5 (mW/cm²) at a pulse width of 837 ns, the UV irradiance is 4.6 (mW/cm²) at a pulse width of 785 ns, the UV irradiance is 4.9 (mW/cm²) at a pulse width of 732 ns, the UV irradiance is 5.0 (mW/cm²) at a pulse width of 695 ns, and the UV irradiance is 5.0 (mW/cm²) at a pulse width of 646 ns.

As a result, it can be seen that the UV irradiance value rapidly changes when the pulse voltage width is around 750 ns ~ 800 ns. Therefore, it can be seen that the UV irradiance value is as high as 4.5 (mW/cm²) when the pulse voltage width is 800 ns or less, and the UV irradiance value is as high as 4.8 (mW/cm²) when the pulse voltage width is 750 ns or less.

As described above, regarding the relationship between the pulse voltage width and the UV irradiance value, when the pulse voltage width is small, the change in voltage becomes rapid, the impedance of the quartz glass (dielectric material) constituting the light-emitting tube decreases, and the potential loss in the quartz glass decreases. Therefore, it can be presumed that the voltage can be efficiently supplied into the discharge space and the efficiency increases accordingly.

Substantially the same results as the experimental results illustrated in Figs. 5 and 6 can be obtained in the discharge lamp illustrated in Figs. 2 and 3 at least in a case where the total length of the lamp is 100 mm or less, even if some design specifications are different.

Note that, in the configuration of the light source device illustrated in Fig. 1, a plurality of the lamps 1, for example, four lamps 1 can be connected in parallel. In this case, the voltage waveform generated in the secondary winding of the transformer can be equally supplied to the plurality of lamps.

Here, the present invention is characterized by employing the flyback circuit. Generally, in a lighting device for a discharge lamp using dielectric barrier discharge, a voltage of a sine wave or a rectangular pulse wave is often supplied to the lamp. This is because a conventionally known discharge lamp using dielectric barrier discharge generally has a large total length of 100 mm or more, and a voltage having a high KV level is required in order to stably generate discharge between electrodes. On the other hand, in the light source device according to the present invention, in order to respond to the demand for miniaturization, the lamp is also miniaturized, and the flyback lighting circuit is adopted. As a numerical example, the rated lamp power is several tens of W, for example, 20 W or less.

The light source device according to the present invention includes: the ultraviolet radiation lamp that emits light by dielectric barrier discharge; and the lighting circuit of the ultraviolet radiation lamp, in which the ultraviolet radiation lamp includes the light-emitting tube having a substantially rod-shape and in which light-emitting gas containing at least a halogen gas is sealed, and the pair of electrodes for generating discharge plasma inside the light-emitting tube in a direction in which the light-emitting tube extends. Further, the lighting circuit can stabilize the discharge column generated between the pair of electrodes of the lamp by constituting the flyback circuit and controlling the width of the pulse voltage applied to the pair of electrodes to be 1000 ns or less.

Furthermore, the light source device according to the present invention can increase the UV irradiance value by controlling the width of the pulse voltage applied to the pair of electrodes to 800 ns or less.

Furthermore, in the light source device according to the present invention, the ultraviolet radiation lamp can emit UVC light having a spectrum exhibiting a peak value at a wavelength of 222 nm by having krypton and chlorine sealed therein, and can emit UVC light having a spectrum exhibiting a peak value at a wavelength of 207 nm by having krypton and bromine sealed therein.

Furthermore, by the light source device according to the present invention being provided with a filter that cuts off radiation light of UVC other than light having a wavelength of 200 nm ~ 230 nm, viruses can be inactivated and bacteria can be killed, and meanwhile, influence on the human body can be more reliably avoided.

Note that, in the light source device according to the present invention, when the width of the pulse voltage applied to the pair of electrodes is controlled to be too narrow, the lighting operation of the lamp becomes difficult. The ultraviolet radiation lamp according to the present invention includes the light-emitting tube in which light-emitting gas containing at least a halogen gas is sealed, and is specifically a KrCI excimer lamp in which krypton and chlorine are sealed, or a KrBr excimer lamp in which krypton and bromine are sealed. In the ultraviolet radiation lamp, when the startability is poor and the pulse voltage width becomes too narrow, power supply to the lamp becomes insufficient, and in some cases, the lamp is not turned on easily.

Specifically, when the applied pulse width becomes too narrow, a pulse voltage value that can be applied is greatly reduced due to the influence of parasitic elements in the power supply line and the light-emitting tube, and the lighting operation of the lamp becomes difficult. Therefore, the light source device according to the present embodiment is controlled so as to secure a pulse voltage width of 450 nanoseconds or more. Note that, in order to realize more reliable lighting, a width of 500 nanoseconds or more is desirably secured as the pulse voltage width (half value width), a width of 550 nanoseconds or more is more desirably secured, and a width of 600 nanoseconds or more is particularly desirably secured.

### DESCRIPTION OF REFERENCE SIGNS

- 1: Excimer lamp
- 2: Transformer
- 3: Input circuit
- 4: Switching element
- 5: Drive circuit

## Claims

1. A light source device comprising:
an ultraviolet radiation lamp that emits light by dielectric barrier discharge; and a lighting circuit of the ultraviolet radiation lamp, wherein
the ultraviolet radiation lamp includes a light-emitting tube having a substantially rod-shape and in which light-emitting gas containing at least a halogen gas is sealed, and a pair of electrodes for generating discharge plasma inside the light-emitting tube in a direction in which the light-emitting tube extends, and
the lighting circuit constitutes a flyback circuit and controls a width of pulse voltage applied to the pair of electrodes to be 450 ns or more to 1000 ns or less.

2. The light source device according to claim 1, wherein the lighting circuit controls the width of the pulse voltage applied to the pair of electrodes to be 800 ns or less.

3. The light source device according to claim 1, wherein the ultraviolet radiation lamp has krypton and chlorine sealed inside, or has krypton and bromine sealed inside.

4. The light source device according to claim 1 that inactivates viruses and kills bacteria, wherein the ultraviolet radiation lamp has a filter that emits light having a wavelength of 200 nm ~ 230 nm and cuts off radiation light of UVC having other wavelengths.
